# EUROPEAN PATENT SPECIFICATION

(11) **EP 4 325 431 B1**
(45) Date of publication and mention of the grant of the patent: **06.11.2024**
(21) Application number: 22190410.5
(22) Date of filing: 15.08.2022
(51) Int. Cl.: G06T 7/11, G06T 7/00

(54) **PROSTATE CANCER LOCAL STAGING**
LOKALE STADIENBESTIMMUNG BEI PROSTATAKREBS
STADIFICATION LOCALE DU CANCER DE LA PROSTATE

(43) Date of publication of application: 21.02.2024
(73) Proprietor: Bayer AG, 51373 Leverkusen (DE)
(72) Inventor: LORIO, Sara, RG4 5DQ Reading (GB); URBAN, Katharina, 15732 Eichwalde (DE)
(74) Representative: BIP Patents

(56) References cited:
- OSCAR J PELLICER-VALERO ET AL: "Deep Learning for fully automatic detection, segmentation, and Gleason Grade estimation of prostate cancer in multiparametric Magnetic Resonance Images", ARXIV.ORG, CORNELL UNIVERSITY LIBRARY, 201 OLIN LIBRARY CORNELL UNIVERSITY ITHACA, NY 14853, 2 February 2022 (2022-02-02), XP091144098
- VENTE COEN DE ET AL: "Deep Learning Regression for Prostate Cancer Detection and Grading in Bi-Parametric MRI", IEEE TRANSACTIONS ON BIOMEDICAL ENGINEERING, IEEE, USA, vol. 68, no. 2, 8 May 2020 (2020-05-08), pages 374 - 383, XP011832049, ISSN: 0018-9294, [retrieved on 20210120], DOI: 10.1109/TBME.2020.2993528
- BELUE MASON J. ET AL: "Tasks for artificial intelligence in prostate MRI", EUROPEAN RADIOLOGY EXPERIMENTAL, vol. 6, no. 1, 6 February 2022 (2022-02-06), XP093020642, Retrieved from the Internet <URL:https://eurradiolexp.springeropen.com/counter/pdf/10.1186/s41747-022-00287-9.pdf> DOI: 10.1186/s41747-022-00287-9
- MOROIANU STEFANIA L. ET AL: "Computational Detection of Extraprostatic Extension of Prostate Cancer on Multiparametric MRI Using Deep Learning", CANCERS, vol. 14, no. 12, 7 June 2022 (2022-06-07), CH, pages 2821, XP093020646, ISSN: 2072-6694, DOI: 10.3390/cancers14122821

## Description

### FIELD

Systems, methods, and computer programs disclosed herein relate to prostate cancer local staging based on multi-parametric magnetic resonance imaging images using a trained machine learning model.

### BACKGROUND

Prostate cancer staging is the process by which physicians categorize the risk of cancer having spread beyond the prostate. The presence of locally advanced prostate cancer, in the form of extracapsular extension, seminal vesicle invasion, or regional lymph node metastasis, can affect the choice of treatment. Accurate local staging of prostate cancer is critical for cancer treatment and patient management decisions.

Once patients are placed in prognostic categories, this information can contribute to the selection of an optimal approach to treatment. Prostate cancer stage can be assessed by imaging techniques or pathological specimens. Clinical staging usually occurs before the first treatment and tumor presence is determined through imaging and needle biopsy, while pathological grading is done after a biopsy is performed or the prostate is removed by looking at the cell types within the sample.

Multi-parametric magnetic resonance imaging (mpMRI) of the prostate is a novel promising tool for diagnosis of prostate cancer (A. Stabile et al.: Multiparametric MRI for prostate cancer diagnosis: current status and future directions, Nat Rev Urol 2020, 17: 41-61).

mpMRI is recommended for local staging of prostate cancer (I. Caglic et al.: Multiparametric MRI - local staging of prostate cancer and beyond, Radiol Oncol. 2019, 53(2):159-170).

Machine learning methods are used to detect and classify prostate cancer based on mpMRI images (EP3367331A1).

O. J. Pellicer-Valero *et al.* disclose a fully automatic system based on Deep Learning that performs localization, segmentation and Gleason grade group (GGG) estimation of prostate cancer lesions from prostate mpMRIs (O. J. Pellicer-Valero et al.: Deep learning for fully automatic detection, segmentation, and Gleason grade estimation of prostate cancer in multiparametric magnetic resonance images, Sci Rep 12, 2975 (2022)).

C. de Vente *et al.* disclose a neural network that simultaneously detects and grades prostate cancer tissue in an end-to-end fashion (C. de Vente et al.: Deep Learning Regression for Prostate Cancer Detection and Grading in Bi-Parametric MRI, IEEE Trans Biomed Eng. 2021 Feb;68(2):374-383).

M. J. Belue and B. Turkbey provide an overview of artificial intelligence (AI) models for prostate segmentation, anatomically segmenting cancer suspicious foci, detecting and differentiating clinically insignificant cancers from clinically significant cancers on a voxel-level, and classifying entire lesions into Prostate Imaging Reporting and Data System categories/Gleason scores (M. J. Belue and B. Turkbey: Tasks for artificial intelligence in prostate MRI, Eur Radiol Exp 6, 33 (2022)).

S. L. Moroianu *et al.:* disclose a method for computational detection of extraprostatic extension on multiparametric MRI using deep learning (S. L. Moroianu et al.: Computational Detection of Extraprostatic Extension of Prostate Cancer on Multiparametric MRI Using Deep Learning, Cancers (Basel), 2022 Jun 7;14(12):2821).

Based on the described state of the art, the technical task was to support physicians in local staging prostate cancer and to burden the patient as little as possible with surgical interventions without increasing the risk of misdiagnosis.

### SUMMARY

This task is solved by the objects of the independent patent claims. Preferred embodiments can be found in the dependent patent claims, the present description, and the drawings.

Therefore, in a first aspect, the present disclosure provides a computer-implemented method for predicting a prostate cancer local stage for a new patient using the trained machine learning model. The prediction method comprises:
- receiving patient data, the patient data comprising a multi-parametric MRI image set of an examination region comprising a prostate region of the new patient,
- inputting the patient data into the trained machine learning model, wherein the trained machine learning model is configured and trained on training data to
   ∘ segment prostatic and extra-prostatic cancer lesions and generate one or more segmented images,
   ∘ assign the one or more segmented images to one of at least two classes, wherein each class corresponds to a prostate cancer local stage,
- receiving from the trained machine learning model a predicted prostate cancer local stage and optionally the one or more segmented images for the new patient,
- outputting the predicted prostate cancer local stage and optionally the one or more segmented images, and/or storing the predicted prostate cancer local stage and optionally the one or more segmented images on a data storage, and/or transmitting the predicted prostate cancer local stage and optionally the one or more segmented images to a remote computer system,
characterized in that the machine learning model comprises a first segmentation unit and a second segmentation unit,
- wherein the first segmentation unit is configured and trained to receive a multi-parametric MRI image set of the examination region and to generate one or more first segmented images based on the multi-parametric MRI image set, wherein the prostate gland, if present is segmented in the one or more first segmented images,
- wherein the second segmentation unit is configured and trained to receive the one or more first segmented images and the multi-parametric MRI image set of the examination region and to generate one or more second segmented images based on the first segmented images and the multi-parametric MRI image set, wherein the prostatic cancer lesions and extra-prostatic cancer lesions, if present, are segmented in the one or more second segmented images,
the method comprising:
- inputting the multi-parametric MRI image set into the first segmentation unit,
- receiving one or more first segmented images from the first segmentation unit,
- inputting the multi-parametric MRI image set and the first segmented images into the second segmentation unit,
- receiving one or more second segmented images from the second segmentation unit,
- inputting the one or more second segmented images into the classification unit,
- receiving from the classification unit a predicted prostate cancer local stage,
- outputting the predicted prostate cancer local stage and optionally the one or more first and/or second segmented images, and/or storing the predicted prostate cancer local stage and optionally the one or more first and/or second segmented images on a data storage, and/or transmitting the predicted prostate cancer local stage and optionally the one or more first and/or second segmented images to a remote computer system.

In another aspect, the present disclosure provides a computer system comprising:
a processor; and a memory storing an application program configured to perform, when executed by the processor, an operation, the operation comprising:
   - receiving patient data, the patient data comprising a multi-parametric MRI image set of an examination region comprising a prostate region of the new patient,
   - inputting the patient data into the trained machine learning model, wherein the trained machine learning model is configured and trained on training data to
      ∘ segment prostatic and extra-prostatic cancer lesions and generate one or more segmented images,
      ∘ assign the one or more segmented images to one of at least two classes, wherein each class corresponds to a prostate cancer local stage,
   - receiving from the trained machine learning model a predicted prostate cancer local stage and optionally the one or more segmented images for the new patient,
   - outputting the predicted prostate cancer local stage and optionally the one or more segmented images, and/or storing the predicted prostate cancer local stage and optionally the one or more segmented images on a data storage, and/or transmitting the predicted prostate cancer local stage and optionally the one or more segmented images to a remote computer system,
   wherein the machine learning model comprises a first segmentation unit and a second segmentation unit,
   - wherein the first segmentation unit is configured and trained to receive a multi-parametric MRI image set of the examination region and to generate one or more first segmented images based on the multi-parametric MRI image set, wherein the prostate gland, if present is segmented in the one or more first segmented images,
   - wherein the second segmentation unit is configured and trained to receive the one or more first segmented images and the multi-parametric MRI image set of the examination region and to generate one or more second segmented images based on the first segmented images and the multi-parametric MRI image set, wherein the prostatic cancer lesions and extra-prostatic cancer lesions, if present, are segmented in the one or more second segmented images,
the operation comprising:
   - inputting the multi-parametric MRI image set into the first segmentation unit,
   - receiving one or more first segmented images from the first segmentation unit,
   - inputting the multi-parametric MRI image set and the first segmented images into the second segmentation unit,
   - receiving one or more second segmented images from the second segmentation unit,
   - inputting the one or more second segmented images into the classification unit,
   - receiving from the classification unit a predicted prostate cancer local stage,
   - outputting the predicted prostate cancer local stage and optionally the one or more first and/or second segmented images, and/or storing the predicted prostate cancer local stage and optionally the one or more first and/or second segmented images on a data storage, and/or transmitting the predicted prostate cancer local stage and optionally the one or more first and/or second segmented images to a remote computer system.

In another aspect, the present disclosure provides a non-transitory computer readable medium having stored thereon software instructions that, when executed by a processor of a computer system, cause the computer system to execute the following steps:
- receiving patient data, the patient data comprising a multi-parametric MRI image set of an examination region comprising a prostate region of the new patient,
- inputting the patient data into the trained machine learning model, wherein the trained machine learning model is configured and trained on training data to
   ∘ segment prostatic and extra-prostatic cancer lesions and generate one or more segmented images,
   ∘ assign the one or more segmented images to one of at least two classes, wherein each class corresponds to a prostate cancer local stage,
- receiving from the trained machine learning model a predicted prostate cancer local stage and optionally the one or more segmented images for the new patient,
- outputting the predicted prostate cancer local stage and optionally the one or more segmented images, and/or storing the predicted prostate cancer local stage and optionally the one or more segmented images on a data storage, and/or transmitting the predicted prostate cancer local stage and optionally the one or more segmented images to a remote computer system,
wherein that the machine learning model comprises a first segmentation unit and a second segmentation unit,
- wherein the first segmentation unit is configured and trained to receive a multi-parametric MRI image set of the examination region and to generate one or more first segmented images based on the multi-parametric MRI image set, wherein the prostate gland, if present is segmented in the one or more first segmented images,
- wherein the second segmentation unit is configured and trained to receive the one or more first segmented images and the multi-parametric MRI image set of the examination region and to generate one or more second segmented images based on the first segmented images and the multi-parametric MRI image set, wherein the prostatic cancer lesions and extra-prostatic cancer lesions, if present, are segmented in the one or more second segmented images,
wherein the software instructions cause the computer system to execute the following steps:
- inputting the multi-parametric MRI image set into the first segmentation unit,
- receiving one or more first segmented images from the first segmentation unit,
- inputting the multi-parametric MRI image set and the first segmented images into the second segmentation unit,
- receiving one or more second segmented images from the second segmentation unit,
- inputting the one or more second segmented images into the classification unit,
- receiving from the classification unit a predicted prostate cancer local stage,
- outputting the predicted prostate cancer local stage and optionally the one or more first and/or second segmented images, and/or storing the predicted prostate cancer local stage and optionally the one or more first and/or second segmented images on a data storage, and/or transmitting the predicted prostate cancer local stage and optionally the one or more first and/or second segmented images to a remote computer system.

### DETAILED DESCRIPTION

The invention will be more particularly elucidated below without distinguishing between the aspects of the disclosure (prediction method, computer system, computer-readable storage medium). On the contrary, the following elucidations are intended to apply analogously to all the aspects of the disclosure, irrespective of in which context (prediction method, computer system, computer-readable storage medium) they occur.

If steps are stated in an order in the present description or in the claims, this does not necessarily mean that the disclosure is restricted to the stated order. On the contrary, it is conceivable that the steps can also be executed in a different order or else in parallel to one another, unless one step builds upon another step, this absolutely requiring that the building step be executed subsequently (this being, however, clear in the individual case). The stated orders are thus preferred embodiments of the invention.

As used herein, the articles "a" and "an" are intended to include one or more items and may be used interchangeably with "one or more" and "at least one." As used in the specification and the claims, the singular form of "a", "an", and "the" include plural referents, unless the context clearly dictates otherwise. Where only one item is intended, the term "one" or similar language is used. Also, as used herein, the terms "has", "have", "having", or the like are intended to be open-ended terms. Further, the phrase "based on" is intended to mean "based at least partially on" unless explicitly stated otherwise.

Some implementations of the present disclosure will be described more fully hereinafter with reference to the accompanying drawings, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art.

The present disclosure provides means for predicting a prostate cancer local stage for a patient. The prediction is made using a trained machine learning model.

Such a "machine learning model", as used herein, may be understood as a computer implemented data processing architecture. The machine learning model can receive input data and provide output data based on that input data and on parameters of the machine learning model. The machine learning model can learn a relation between input data and output data through training. In training, parameters of the machine learning model may be adjusted in order to provide a desired output for a given input.

The process of training a machine learning model involves providing a machine learning algorithm (that is the learning algorithm) with training data to learn from. The term "trained machine learning model" refers to the model artifact that is created by the training process. The training data must contain the correct answer, which is referred to as the target. The learning algorithm finds patterns in the training data that map input data to the target, and it outputs a trained machine learning model that captures these patterns.

In the training process, training data are inputted into the machine learning model and the machine learning model generates an output. The output is compared with the (known) target. Parameters of the machine learning model are modified in order to reduce the deviations between the output and the (known) target to a (defined) minimum.

In general, a loss function can be used for training, where the loss function can quantify the deviations between the output and the target. The loss function may be chosen in such a way that it rewards a wanted relation between output and target and/or penalizes an unwanted relation between an output and a target. Such a relation can be, e.g., a similarity, or a dissimilarity, or another relation.

If, for example, the output and the target are numbers, the loss function could be the difference between these numbers. In this case, a high absolute value of the loss function can mean that a parameter of the model needs to undergo a strong change.

In the case of vector-valued outputs, for example, difference metrics between vectors such as the root mean square error, a cosine distance, a norm of the difference vector such as a Euclidean distance, a Chebyshev distance, an Lp-norm of a difference vector, a weighted norm or any other type of difference metric of two vectors can be chosen. These two vectors may for example be the desired output (target) and the actual output.

In the case of higher dimensional outputs, such as two-dimensional, three-dimensional or higher-dimensional outputs, for example an element-wise difference metric can be used. Alternatively or additionally, the output data may be transformed, for example to a one-dimensional vector, before computing a loss.

The modification of model parameters and the reduction of the loss can be done in an optimization procedure, for example in a gradient descent procedure.

The machine learning model of the present disclosure comprises at least one segmentation unit. The at least one segmentation unit is configured and trained to receive a multi-parametric MRI image set of an examination region comprising a prostate region of a male human and to generate one or more segmented images based on the one or more received images and model parameters. In the one or more segmented images prostatic and extra-prostatic cancer lesions, if present, are segmented.

The term "segmentation" refers to the process of dividing an image into several segments, also known as image segments, image regions or image objects. Segmentation is typically used to locate objects and boundaries (lines, curves, etc.) in images. From a segmented image, the localized objects can be separated from the background, visually highlighted (e.g.: colored), measured, counted, or otherwise quantified.

Segmentation involves assigning a label to each pixel/voxel of an image such that pixels/voxels with the same label have certain features in common.

For example, in a segmented image, where the prostate gland is segmented, all pixels/voxels representing the prostate gland have the same label. In a segmented image, where prostatic cancer lesions are segmented all pixels/voxels representing prostatic cancer lesions have the same label. In a segmented image, where extra-prostatic cancer lesions are segmented all pixels/voxels representing extra-prostatic cancer lesions have the same label.

For example, in the present case, a 4-class segmentation mask may be generated in which different labels are assigned to the pixels/voxels representing the prostate gland, a prostatic cancer lesion, an extra-prostatic cancer lesion, and any other tissue (background) (e.g., class 0 = background, class 1 = prostate gland, class 2 = prostatic cancer lesion, class 3 = extra-prostatic cancer lesion).

For example, in the present case, a 3-class segmentation mask may be generated in which different labels are assigned to the pixels/voxels representing a prostatic cancer lesion, an extra-prostatic cancer lesion, and any other tissue (background) (e.g., class 0 = background, class 1 = prostatic cancer lesion, class 2 = extra-prostatic cancer lesion).

Segmentation can be done in stages. Segmentation can be done, e.g., in two steps: in a first step, the prostate gland can be segmented; in a second step, prostatic and extra-prostatic cancer lesions can be segmented. Segmentation can be done, e.g., in three steps: in a first step, the prostate gland can be segmented; in a second step, prostatic cancer lesions can be segmented; in a third step, extra-prostatic cancer lesions can be segmented. However, the segmentation can also be done in one step.

In a preferred embodiment, the machine learning model comprises two segmentation units, a first segmentation unit and a second segmentation unit.

The first segmentation unit is configured and trained to receive a multi-parametric MRI image set of an examination region comprising a prostate region of a male human and to generate one or more segmented images based on the one or more received images in which the prostate gland of the male human, if present, is segmented.

The one or more segmented images generated by the first segmentation unit can be a mask and/or a mask can be created therefrom. A "mask" is an image where pixel/voxel intensity values are zero, and others are non-zero. A prostate gland mask may be an image where intensity values of pixels/voxels representing extra-prostatic tissue are set to zero. An extra-prostatic region mask may be an image where intensity values of pixels/voxels representing a prostate gland are set to zero. Such masks can be used for masking other images: wherever the pixel/voxel intensity value is zero in the mask, then the pixel intensity of the resulting masked image will be set to zero. The prostate gland mask and/or the extra-prostatic region mask can be used to mask the multi-parametric MRI image set. They can be used to create an image set that is reduced to the prostate gland and/or they can be used to create an image set that is reduced to extra-prostatic regions. In this way, in a further step in which lesions are identified and segmented, it can be determined whether the lesions are located inside or outside the prostate.

The second segmentation unit can be configured and trained to receive the multi-parametric MRI image set of the male human and the one or more segmented images in which the prostate gland of the male human is segmented (and/or one or more masks created therefrom), and to generate one or more segmented images in which prostatic and extra-prostatic cancer lesions are segmented.

The one or more segmented images generated by the second segmentation model can then be used for classification (prediction of the prostate cancer local stage).

For this purpose, the machine learning model of the present disclosure comprises a classification unit. The classification unit is configured to assign the one or more segmented images to one of at least two classes, each class corresponding to a prostate cancer local stage.

The machine learning model of the present disclosure is trained on training data.

The training data comprise, for each patient of a multitude of patients, input data and target data. The term "multitude of patients" means at least 10, preferably at least 100 patients.

Patients usually include patients with prostate cancer, patients with suspected prostate cancer, patients who have had their prostates removed due to prostate cancer, and/or healthy patients. Patients usually include those with various stages of prostate cancer. Usually, patients cover all stages that the machine learning model is trained to predict.

The input data comprise a multi-parametric MRI image set of an examination region comprising the prostate of the patient.

The multi-parametric MRI image set may include different types of MRI images and/or maps.

Each image and/or map is a representation of an examination region of a patient. The examination region usually comprises the prostate of the patient, or, in case of patients who have had the prostate removed, the region where the prostate was located prior to prostatectomy, and surrounding tissue, hereinafter collectively referred to as the prostate region.

The multi-parametric MRI (mpMRI) image set refers to a plurality of MRI images/maps acquired using various different MRI acquisition/image generation techniques. The different image channels acquired using different imaging techniques provide different information at locations in the prostate region of the patient. Corresponding pixel/voxel locations in the different image channels refer to the same location in the prostate region, and each pixel/voxel location has a vector of image values including a respective image value for each image channel.

In an advantageous embodiment, the mpMRI image set of the prostate region of a patient is a set of 2D, 3D or 4D (e.g., 3D plus time) mpMRI images and/or maps.

Any or all of the images and/or maps in the mpMRI image set of the patient's prostate region can be received directly from an MRI scanner used to acquire mpMRI images and/or maps. Alternatively, any or all of the images and/or maps in the mpMRI image set of the patient's prostate region can be provided by loading/retrieving previously acquired images and/or maps from a storage or memory of a computer system or receiving previously acquired images and/or maps via an electronic transmission from a remote computer system.

In an advantageous embodiment, the mpMRI image set includes a T2-weighted pulse sequence (T2-weighted MRI image) that provides an overview of the prostate region.

The mpMRI image set can also include functional imaging, such as one or more diffusion weighted imaging (DWI) image depicting water molecule diffusion variations due to the microscopic tissue structures. DWI generates a set of images using different gradients (or b-values), which result in different reconstructed signal intensities.

The mpMRI image set can also include an apparent diffusion coefficient (ADC) map which can be generated from the DWI image set. The ADC map can be derived using the signal intensity changes of at least two b-values and provides a quantitative map demonstrating the degree of water molecule diffusion.

Additionally, dynamic contrast enhanced (DCE) MRI can be included in the overall acquisition of the mpMRI image set. In DCE MRI, a series of temporally fast T1-weighted MRI images are acquired during rapid intravenous injection of a gadolinium-based contrast agent.

Prostate cancer tissues often induce some level of angiogenesis, which is followed by an increased vascular permeability as compared to normal prostatic tissue. A *K^{trans}* map can be generated from the DCE MRI image set and included in the mpMRI image set. *K^{trans}* is a measure that provides an indicator of tissue permeability.

mpMRI image sets can be obtained using a 1.5T or preferably a 3T scanner with or without an endo-rectal coil. Examples of MR imaging protocols are given, e.g., in I. Caglic et al.: Multiparametric MRl - local staging of prostate cancer and beyond, Radiol Oncol. 2019, 53(2): 159-170.

Before the images and/or maps of an mpMRI image set are inputted into the machine learning model, they can be pre-processed.

Pre-processing usually comprise motion compensation and/or region-of-interest extraction. Prior to inputting the mpMRI image set into the machine learning model, the motion compensation can be performed on the mpMRI image set to compensate for any motion (e.g., patient movement) between the various MRI acquisitions (e.g., T2-weighted, DWI, DCE). In an advantageous implementation, 3D elastic registration is used in a cascade fashion to perform the motion compensation. In order to increase robustness, a pairwise registration can be performed between the T2-weighted MRI image and a corresponding low b-value image in the DWI image set, resulting in a computed deformation field. The computed deformation field can then be applied to compensate motion in the ADC parameter map. The computed deformation field can also be applied to compensate motion in other images of the DWI image set, such as a high b-value DWI image. Similarly, a pairwise registration can be performed between the T2-weighted MR image and a late contrast-enhanced image representative of the DCE MRI image set, and the resulting computed deformation field can be applied to perform motion compensation in the *K^{Trans}* map.

In case the MRI scans used to acquire the mpMRI image set cover an area larger than the prostate region, a region-of-interest (ROI) surrounding the prostate can be extracted in the mpMRI image set. A predetermined size ROI mask can be applied to each slice of the images in the mpMRI image set to ensure that only the prostate and surrounding area in each image is considered for the prostate cancer stage prediction. For example, an 80 mm x 80 mm ROI mask can be applied to each slice. After the motion compensation and/or ROI extraction, the images in the mpMRI image set may then be reformatted into T2-weighted image grid with a predetermined size that corresponds to size of the input channels of the machine learning model. For example, each image and/or map can be reformatted into a T2-weighted image grid with the size of 100 mm x 100 mm x 60 mm, which corresponds to roughly 200 x 200 pixels in each 2D slice.

Besides the mpMRI image set of a patient, the input data can comprise further patient data such as patient's age, body size, body weight, body mass index, prostate-specific antigen (PSA) level, biopsy Gleason score, resting heart rate, heart rate variability, body temperature, lifestyle information about the life of the patient, such as consumption of alcohol, smoking, and/or exercise and/or the patient's diet, information about a previous prostatectomy, medical intervention parameters such as regular medication, occasional medication, or other previous or current medical interventions and/or other information about the patient's previous and current treatments and reported health conditions and/or combinations thereof.

The training data further comprise, for each patient of the plurality of patients, one or more MRI images in which, if present, prostate gland, prostatic and extra-prostatic cancer lesions are segmented. The segmentation can be done manually by a radiologist, for example. The one or more (manually) segmented images serve as target data for training the at least one segmentation unit.

The training data further comprise, for each patient of the plurality of patients, a prostate cancer local stage as target data for training the classification unit.

There are different systems for staging (prostate) cancer. The machine learning model can in principle be trained to predict the staging according to each system. The machine learning model can also be trained to predict staging according to different (more than one) systems.

The most widely used system for staging of prostate cancer is the tumor, nodes, and metastases (TNM) staging system developed by the American Joint Committee on Cancer (AJCC). In a preferred embodiment, the machine learning model is trained to make a prediction in accordance with the TNM staging system. Accordingly, the training data contains a TNM stage for each patient.

It is also possible to have only two stages, for example, one stage indicating that no tumor is present or that an existing tumor has not spread beyond the prostate, and a second stage indicating that the tumor has spread beyond the prostate.

The at least one segmentation unit and the classification unit can be trained jointly or separately from each other. Preferably, the at least one segmentation unit and the classification unit are trained separately from each other.

If the at least one segmentation unit comprises two or more segmentation units, each segmentation unit can be trained separately, or two or more segmentation unit can be trained jointly. Preferably, each segmentation unit is trained separately.

During training of a segmentation unit, one or more input images to be segmented are inputted into the segmentation unit and the segmentation unit generates one or more segmented images based on the input image(s) and model parameters. The one or more segmented images are compared with respective target image(s) and deviations between input image(s) and target image(s) can be quantified using a loss function. Model parameters can then be modified to reduce the segmentation loss.

During training of the classification unit, one or more segmented images are inputted into the classification unit and the classification assigns the one or more segmented images to one of at least two classes based on the inputted image(s) and model parameters, wherein each class corresponding to a prostate cancer local stage. The class to which the one or more segmented images is/are assigned is compared with the target prostate cancer local stage and deviations between the class and the target prostate cancer local stage can be quantified using a loss function. Model parameters can then be modified to reduce the classification loss.

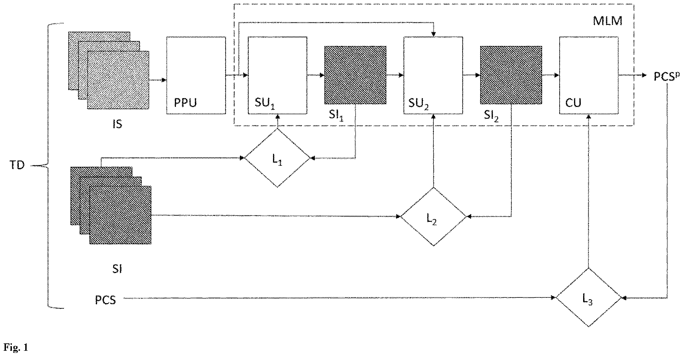

The training procedure is schematically depicted in Fig. 1 for the example of a machine learning model comprising two segmentation units, SU 1 and SU2, and a classification unit CU.

The machine learning model MLM is trained with training data. The training data comprise data from a multitude of patients. In Fig. 1 only one set of training data TD of one patient is shown, the training data TD comprising an mpMRI image set IS of the prostate region of the patient, one or more segmented images in which the prostate gland as well as prostatic and extra-prostatic lesions, if present, are segmented, and a prostate cancer stage PCS.

In the example depicted in Fig. 1, the mpMRI image set IS is inputted into a pre-processing unit PPU. The pre-processing unit PPU is configured to perform motion compensation and/or region-of-interest extraction.

The preprocessed images are then inputted into the first segmentation unit SU₁. The first segmentation unit SU₁ is configured to generate one or more first segmented images SI₁ in which the prostate gland, if present, is segmented. Deviations between the one or more first segmented images SI₁ and the respective one or more segmented images SI of the training data (target data) are quantified by means of a first loss function L₁. The calculated loss can be used to modify model parameters of the first segmentation unit SU₁ to reduce the deviations, e.g., to a pre-defined minimum. The dice loss function can be used as a loss function (see, e.g., S. Jadon: A survey of loss functions for semantic segmentation, arXiv:2006.14822v4 [eess.IV] 3 Sep 2020).

The one or more first segmented images SI₁ together with the preprocessed images are then inputted into the second segmentation unit SU₂. The second segmentation unit SU₂ is configured to generate one or more second segmented images SI₂ in which prostatic and extra-prostatic lesions, if present, are segmented. Deviations between the one or more second segmented images SI₂ and the respective one or more segmented images SI of the training data (target data) are quantified by means of a second loss function L₂. The calculated loss can be used to modify model parameters of the second segmentation unit SU₂ to reduce the deviations, e.g., to a pre-defined minimum. The dice loss function can be used as a loss function.

The one or more second segmented images SI₂ are then inputted into the classification unit CU. The classification unit CU is configured to assign the one or more second segmented images SI₂ to one of at least two classes, each class corresponding to a prostate cancer local stage. Deviations between the class the one or more second segmented images SI₂ are assigned to (the predicted prostate cancer local stage PCS^{P}) and the prostate cancer local stage PCS of the training data (target data) are quantified by means of a third loss function L₃. The calculated loss can be used to modify model parameters of the classification unit CU to reduce the deviations, e.g., to a pre-defined minimum. Examples of loss functions include L1 loss, L2 loss, structure similarity index measure (SSIM) or combination of the above to name a few. More details about loss functions may be found in the scientific literature (see e.g.: K. Janocha et al.: On Loss Functions for Deep Neural Networks in Classification, 2017, arXiv:1702.05659v1 [cs.LG].

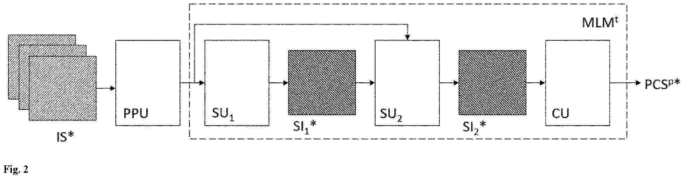

Fig. 2 shows schematically, by way of example, the process of predicting a prostate cancer stage for a new patient using the trained machine learning model MLM^{t}. Patient data comprising an mpMRI image set IS* of the new patient are received. The mpMRI image set IS* is inputted into the pre-processing unit PPU. The pre-processing unit PPU is configured to perform motion compensation and/or region-of-interest extraction. The pre-processed images are then inputted into the first segmentation unit SU₁ that generates one or more first segmented images SI₁* in which the prostate gland, if present, is segmented. The one or more first segmented images SI₁* together with the pre-processed images are then inputted into the second segmentation unit SU₂ that generates one or more second segmented images SI₂* in which prostatic and extra-prostatic lesions, if present, are segmented. The one or more second segmented images SI₂* are then inputted into the classification unit CU. The classification unit outputs a predicted prostate cancer local stage PCS^{P}* for the new patient. The predicted prostate cancer local stage PCS^{P}* for the new patient as well as the one or more first and/or second segmented images SI₁* and/or SI₂* can be displayed on a display, printed on a printer, stored on a data memory and/or transmitted to a remote computer system. The predicted prostate cancer local stage PCS^{P}* for the new patient as well as the one or more first and/or second segmented images SI1* and/or SI2* can be used by a radiologist to determine a therapy for the patient.

The machine learning model can be or comprise one or more artificial neural networks. An artificial neural network is a biologically inspired computational model. Such an artificial neural network usually comprises at least three layers of processing elements: a first layer with input neurons, an nth layer with at least one output neuron, and *n-2* inner layers, where *n* is a natural number greater than 2. In such a network, the input neurons serve to receive the input data. If the input data constitutes or comprises an image, there is usually one input neuron for each pixel/voxel of the input image; there can be additional input neurons for additional input data such as data about the object represented by the input image, the type of image, the way the image was acquired, additional patient data and/or the like. The output neurons serve to output one or more values, e.g., a predicted prostate cancer local stage, a segmented image, and/or others.

The processing elements of the layers are interconnected in a predetermined pattern with predetermined connection weights therebetween. Each network node usually represents a calculation of the weighted sum of inputs from prior nodes and a non-linear output function. The combined calculation of the network nodes relates the inputs to the outputs.

When trained, the connection weights between the processing elements contain information regarding the relationship between the input data and the output data.

Each network node can represent a calculation of the weighted sum of inputs from prior nodes and a non-linear output function. The combined calculation of the network nodes relates the inputs to the outputs.

The network weights can be initialized with small random values or with the weights of a prior partially trained network. The training data inputs are applied to the network and the output values are calculated for each training sample. The network output values can be compared to the target output values. A backpropagation algorithm can be applied to correct the weight values in directions that reduce the error between calculated outputs and targets. The process is iterated until no further reduction in error can be made or until a predefined prediction accuracy has been reached.

A cross-validation method can be employed to split the data into training and validation data sets. The training data set is used in the error backpropagation adjustment of the network weights. The validation data set is used to verify that the trained network generalizes to make good predictions. The best network weight set can be taken as the one that presumably best predicts the outputs of the test data set. Similarly, varying the number of network hidden nodes and determining the network that performs best with the data sets optimizes the number of hidden nodes.

In a preferred embodiment, the one or more artificial neural networks is/are or comprise(s) one or more convolutional neural networks (CNN). A CNN is a class of artificial neural networks, most commonly applied to, e.g., analyzing visual imagery. A CNN comprises an input layer with input neurons, an output layer with at least one output neuron, as well as multiple hidden layers between the input layer and the output layer.

The hidden layers of a CNN typically comprise convolutional layers, ReLU (Rectified Linear Units) layers, i.e. activation function, pooling layers, fully connected layers and normalization layers.

The nodes in the CNN input layer can be organized into a set of "filters" (feature detectors), and the output of each set of filters is propagated to nodes in successive layers of the network. The computations for a CNN include applying the mathematical convolution operation with each filter to produce the output of that filter. Convolution is a specialized kind of mathematical operation performed with two functions to produce a third function. In convolutional network terminology, the first function of the convolution can be referred to as the input, while the second function can be referred to as the convolution kernel. The output may be referred to as the feature map. For example, the input of a convolution layer can be a multidimensional array of data that defines the various color components of an input image. The convolution kernel can be a multidimensional array of parameters, where the parameters are adapted by the training process for the neural network.

The objective of the convolution operation is to extract features (such as, e.g., edges from an input image). Conventionally, the first convolutional layer is responsible for capturing the low-level features such as edges, color, gradient orientation, etc. With added layers, the architecture adapts to the high-level features as well, giving a network which has the wholesome understanding of images in the dataset. Similar to the convolutional layer, the pooling layer is responsible for reducing the spatial size of the feature maps. It is useful for extracting dominant features with some degree of rotational and positional invariance, thus maintaining the process of effectively training of the model. Adding a fully-connected layer is a way of learning non-linear combinations of the high-level features as represented by the output of the convolutional part.

The machine learning model can be configured as a classification model. It can be configured to receive input data comprising a multi-parametric MRI image set of a patient, and to assign the input data to one of several classes, where each class usually corresponds to a prostate cancer stage.

However, the model can also be configured to generate and output one or more segmented images based on the input data.

For example, in the present case, the segmented image(s) may show the prostate, the area where the prostate was located before prostatectomy, the seminal vesicle, invasions into the seminal vesicle, lymph nodes, lymph node metastases, extracapsular extensions, extra-prostatic extension, invasions of the bladder neck, the external sphincter, the rectum, the levator muscles and/or the pelvic wall, and/or other objects segmented.

In the segmented image, features (regions) that have led to the predicted prostate cancer stage can be marked, e.g., by a defined color. For example, if extra-prostatic extensions occur in the multi-parametric MRI image set of a patient leading to a TNM-stage T3 classification, these extra-prostatic extensions can be marked in the at least one segmented image with a defined color representing the TNM-stage T3. In this way, the trained machine learning model performs simultaneous identification of prostate cancer signs and metastases and prostate cancer local staging.

The one or more segmentation units can be one or more generative neural network such as one or more image-to-image convolutional encoder-decoder.

For example, each segmentation unit can be based on a specific kind of convolutional architecture called U-Net (see e.g. O. Ronneberger et al.: U-net: Convolutional networks for biomedical image segmentation, in: International Conference on Medical image computing and computer-assisted intervention, pp. 234-241, Springer, 2015, https://doi.org/10.1007/978-3-319-24574-4_28). The U-Net architecture consists of two main blocks, an encoding path and a decoding path. The encoding path uses convolutions, activation functions and pooling layers to extract image features, while the decoding path replaces the pooling layers with upsampling layers to project the extracted features back to pixel/voxel space, and finally recovers the image dimension at the end of the architecture. These are used in combination with activation functions and convolutions. Finally, the feature maps from the encoding paths can be concatenated to the feature maps in the decoding path in order to preserve fine details from the input data.

For example, each segmentation unit can be or comprise a generative adversarial network (GAN), preferably a Pix2Pix GAN or a cycleGAN (see, e.g., M.-Y. Liu et al.: Generative Adversarial Networks for Image and Video Synthesis: Algorithms and Applications, arXiv:2008.02793; J. Henry et al.: Pix2Pix GAN for Image-to-Image Translation, DOI: 10.13140/RG.2.2.32286.66887; J. Haubold et al.: Contrast agent dose reduction in computed tomography with deep learning using a conditional generative adversarial network, Eur Radiol. 2021, 31(8): 6087-6095, doi: 10.1007/s00330-021-07714-2).

The classification unit can be an image classification model. For example, the classification unit can be based on a 3D ResNet18 architecture (see, e.g.: K. Hara et al.: Learning Spatio-temporal Features with 3D Residual Networks for Action Recognition, 2017, arXiv:1708.07632 [cs.CV]).

Once the machine learning model is trained, the trained machine learning model can be stored on a memory or storage of a computer system and used to a predict prostate cancer stage and/or perform simultaneous identification of prostate cancer signs and metastases and prostate cancer staging in newly received/inputted patient data including an mpMRI image set of a new patient. The term "new" means that the respective data have not been used in the training and/or validation process.

The patient data of the new patient are inputted into the trained machine learning model and the trained machine learning model generates and outputs a predicted prostate cancer stage and optionally one or more segmented images.

The predicted prostate cancer stage and/or the one or more segmented images can be outputted by displaying the prostate cancer stage and/or the one or more segmented images on a display device of a computer system and/or by printing the prostate cancer stage and/or the one or more segmented images via a printing device.

It is possible that the predicted prostate cancer stage is compared with one or more reference values. For example, if the predicted prostate cancer stage is below a reference value, this may mean that prostatectomy is recommended. If the predicted prostate cancer stage is greater than or equal to the reference value, it may mean that chemotherapy is recommended, possibly in combination with prostatectomy.

The operations in accordance with the teachings herein may be performed by at least one computer specially constructed for the desired purposes or general-purpose computer specially configured for the desired purpose by at least one computer program stored in a typically non-transitory computer readable storage medium.

The term "non-transitory" is used herein to exclude transitory, propagating signals or waves, but to otherwise include any volatile or non-volatile computer memory technology suitable to the application.

The term "computer" should be broadly construed to cover any kind of electronic device with data processing capabilities, including, by way of non-limiting example, personal computers, servers, embedded cores, computing system, communication devices, processors (e.g., digital signal processor (DSP)), microcontrollers, field programmable gate array (FPGA), application specific integrated circuit (ASIC), etc.) and other electronic computing devices.

The term "process" as used above is intended to include any type of computation or manipulation or transformation of data represented as physical, e.g., electronic, phenomena which may occur or reside e.g., within registers and/or memories of at least one computer or processor. The term processor includes a single processing unit or a plurality of distributed or remote such units.

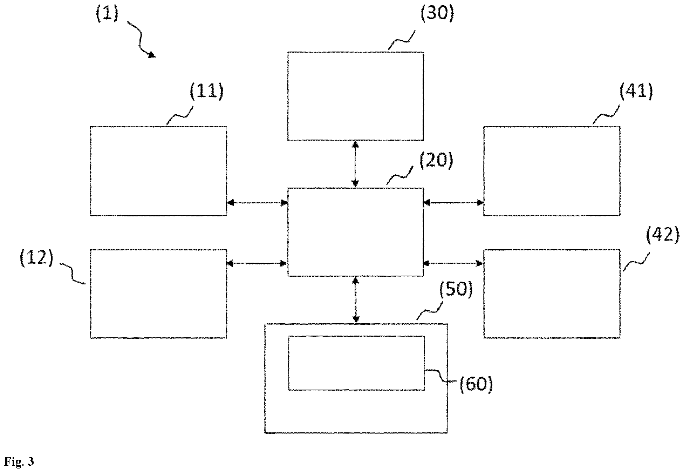

Fig. 3 illustrates a computer system (1) according to some example implementations of the present disclosure in more detail. The computer may include one or more of each of a number of components such as, for example, processing unit (20) connected to a memory (50) (e.g., storage device).

The processing unit (20) may be composed of one or more processors alone or in combination with one or more memories. The processing unit is generally any piece of computer hardware that is capable of processing information such as, for example, data, computer programs and/or other suitable electronic information. The processing unit is composed of a collection of electronic circuits some of which may be packaged as an integrated circuit or multiple interconnected integrated circuits (an integrated circuit at times more commonly referred to as a "chip"). The processing unit may be configured to execute computer programs, which may be stored onboard the processing unit or otherwise stored in the memory (50) of the same or another computer.

The processing unit (20) may be a number of processors, a multi-core processor or some other type of processor, depending on the particular implementation. Further, the processing unit may be implemented using a number of heterogeneous processor systems in which a main processor is present with one or more secondary processors on a single chip. As another illustrative example, the processing unit may be a symmetric multi-processor system containing multiple processors of the same type. In yet another example, the processing unit may be embodied as or otherwise include one or more ASICs, FPGAs or the like. Thus, although the processing unit may be capable of executing a computer program to perform one or more functions, the processing unit of various examples may be capable of performing one or more functions without the aid of a computer program. In either instance, the processing unit may be appropriately programmed to perform functions or operations according to example implementations of the present disclosure.

The memory (50) is generally any piece of computer hardware that is capable of storing information such as, for example, data, computer programs (e.g., computer-readable program code (60)) and/or other suitable information either on a temporary basis and/or a permanent basis. The memory may include volatile and/or non-volatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD, Blu-ray disk or the like. In various instances, the memory may be referred to as a computer-readable storage medium. The computer-readable storage medium is a non-transitory device capable of storing information, and is distinguishable from computer-readable transmission media such as electronic transitory signals capable of carrying information from one location to another. Computer-readable medium as described herein may generally refer to a computer-readable storage medium or computer-readable transmission medium.

In addition to the memory (50), the processing unit (20) may also be connected to one or more interfaces for displaying, transmitting and/or receiving information. The interfaces may include one or more communications interfaces and/or one or more user interfaces. The communications interface(s) may be configured to transmit and/or receive information, such as to and/or from other computer(s), network(s), database(s) or the like. The communications interface may be configured to transmit and/or receive information by physical (wired) and/or wireless communications links. The communications interface(s) may include interface(s) (41) to connect to a network, such as using technologies such as cellular telephone, Wi-Fi, satellite, cable, digital subscriber line (DSL), fiber optics and the like. In some examples, the communications interface(s) may include one or more short-range communications interfaces (42) configured to connect devices using short-range communications technologies such as NFC, RFID, Bluetooth, Bluetooth LE, ZigBee, infrared (e.g., IrDA) or the like.

The user interfaces may include a display (30). The display may be configured to present or otherwise display information to a user, suitable examples of which include a liquid crystal display (LCD), light-emitting diode display (LED), plasma display panel (PDP) or the like. The user input interface(s) (11) may be wired or wireless, and may be configured to receive information from a user into the computer system (1), such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology (12) for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers and the like.

As indicated above, program code instructions may be stored in memory, and executed by processing unit that is thereby programmed, to implement functions of the systems, subsystems, tools and their respective elements described herein. As will be appreciated, any suitable program code instructions may be loaded onto a computer or other programmable apparatus from a computer-readable storage medium to produce a particular machine, such that the particular machine becomes a means for implementing the functions specified herein. These program code instructions may also be stored in a computer-readable storage medium that can direct a computer, processing unit or other programmable apparatus to function in a particular manner to thereby generate a particular machine or particular article of manufacture. The instructions stored in the computer-readable storage medium may produce an article of manufacture, where the article of manufacture becomes a means for implementing functions described herein. The program code instructions may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions may produce a computer-implemented process such that the instructions executed by the computer, processing circuitry or other programmable apparatus provide operations for implementing functions described herein.

Execution of instructions by processing unit, or storage of instructions in a computer-readable storage medium, supports combinations of operations for performing the specified functions. In this manner, a computer system (1) may include processing unit (20) and a computer-readable storage medium or memory (50) coupled to the processing circuitry, where the processing circuitry is configured to execute computer-readable program code (60) stored in the memory. It will also be understood that one or more functions, and combinations of functions, may be implemented by special purpose hardware-based computer systems and/or processing circuitry which perform the specified functions, or combinations of special purpose hardware and program code instructions.

## Claims

1. A computer-implemented method for predicting a prostate cancer local stage for a patient, the prediction method comprising:
- providing a trained machine learning model (MLM^{t}),
- receiving patient data, the patient data comprising a multi-parametric MRI image set (IS*) of an examination region comprising a prostate region of the patient,
- inputting the patient data into the trained machine learning model (MLM'), wherein the trained machine learning model (MLM^{t}) is configured and trained on training data to
∘ segment prostatic and extra-prostatic cancer lesions and generate one or more segmented images (SI₁*, SI₂*) based on the patient data,
∘ assign the one or more segmented images (SI₁*, SI₂*) to one of at least two classes, wherein each class corresponds to a prostate cancer local stage,
- receiving from the trained machine learning model (MLM') a predicted prostate cancer local stage (PCS^{P}*) and optionally the one or more segmented images (SI₁*, SI₂*) for the patient,
- outputting the predicted prostate cancer local stage (PCS^{P}*) and optionally the one or more segmented images (SI₁*, SI₂*), and/or storing the predicted prostate cancer local stage (PCS^{P}*) and optionally the one or more segmented images (SI₁*, SI₂*) on a data storage, and/or transmitting the predicted prostate cancer local stage (PCS^{P}*) and optionally the one or more segmented images (SI₁*, SI₂*) to a remote computer system,
wherein the machine learning model (MLM') comprises a first segmentation unit (SU₁) and a second segmentation unit (SU₂),
- wherein the first segmentation unit (SU₁) is configured and trained to receive the multi-parametric MRI image set (IS*) of the examination region and to generate the one or more first segmented images (SI₁*) based on the multi-parametric MRI image set (IS*), wherein the prostate gland, if present is segmented in the one or more first segmented images (SI₁*),
- wherein the second segmentation unit (SU₂) is configured and trained to receive the one or more first segmented images (SI₁*) and the multi-parametric MRI image set (IS*) of the examination region and to generate the one or more second segmented images (SI₂*) based on the first segmented images (SI₁*) and the multi-parametric MRI image set (IS*), wherein the prostatic cancer lesions and extra-prostatic cancer lesions, if present, are segmented in the one or more second segmented images (SI₂*),
the method comprising:
- inputting the multi-parametric MRI image set (IS*) into the first segmentation unit (SU₁),
- receiving the one or more first segmented images (SI₁*) from the first segmentation unit (SU₁),
- inputting the multi-parametric MRI image set (IS*) and the first segmented images (SI₁*) into the second segmentation unit (SU₂),
- receiving the one or more second segmented images (SI₂*) from the second segmentation unit (SU₂),
- inputting the one or more second segmented images (SI₂*) into a classification unit (CU),
- receiving from the classification unit (CU) the predicted prostate cancer local stage (PCS^{P}*),
- outputting the predicted prostate cancer local stage (PCS^{P}*) and optionally the one or more first and/or second segmented images (SI₁*, SI₂*), and/or storing the predicted prostate cancer local stage (PCS^{P}*) and optionally the one or more first and/or second segmented images (SI₁*, SI₂*) on a data storage, and/or transmitting the predicted prostate cancer local stage (PCS^{P}*) and optionally the one or more first and/or second segmented images (SI₁*, SI₂*) to a remote computer system.

2. The method according to claim 1, wherein the trained machine learning model (MLM^{t}) was trained in a training method, the training method comprising:
- receiving and/or providing a machine learning model (MLM), wherein the machine learning model (MLM) comprises at least one segmentation unit (SU₁. SU₂) and a classification unit (CU), wherein the at least one segmentation unit is (SU₁. SU₂) configured to receive a multi-parametric MRI image set (IS) of an examination region comprising a prostate region of a male human and to generate one or more segmented images (SI₁, SI₂) based on the one or more received images and model parameters, and wherein the classification unit (CU) is configured to assign the one or more segmented images (SI₁, SI₂) to one of at least two classes based on model parameters, each class corresponding to a prostate cancer local stage,
- receiving and/or providing training data (TD), the training data (TD) comprising, for each patient of a multitude of patients, input data and target data, the input data comprising a multi-parametric MRI image set (IS) of an examination region comprising a prostate region of the patient, and the target data comprising one or more target images (SI) in which, if present, prostate gland, prostatic cancer lesions and extra-prostatic cancer lesions are segmented, and a prostate cancer local stage (PCS) for the patient,
- training the machine learning model (MLM), wherein the training comprises for each patient of the multitude of patients:
∘ inputting the multi-parametric MRI image set (IS) of the patient into the at least one segmentation unit (SU₁. SU₂),
∘ receiving one or more segmented images (SI₁. SI₂) from the at least one segmentation unit (SU₁. SU₂) in which, if present, prostatic and extra-prostatic cancer lesions are segmented,
∘ computing a segmentation loss (L₁, L₂), the segmentation loss (L₁, L₂) quantifying deviations between the one or more segmented images (SI₁. SI₂) and the one or more target images (SI),
∘inputting the one or more segmented images (SI₁. SI₂) into the classification unit (CU),
∘ receiving a predicted prostate cancer local stage (PCS^{P}) from the classification unit (CU),
∘ computing a classification loss (L₃), the classification loss (L₃) quantifying deviations between the prostate cancer local stage (PCS) and the predicted prostate cancer local stage (PCS^{P}),
∘ modifying model parameters to reduce the segmentation loss (L₁, L₂) and the classification loss (L₃),
- storing and/or outputting the model parameters and/or the trained machine learning model (MLM^{t}) and/or transmitting the model parameters and/or the trained machine learning model (MLM^{t}) to a remote computer system and/or using the trained machine learning model (MLM^{t}) for predicting a prostate cancer local stage for a new patient.

3. The method according to claim 2,
- wherein the training comprises for each patient of the multitude of patients:
∘ inputting the multi-parametric MRI image set (IS) of the patient into the first segmentation unit (SU₁),
∘ receiving one or more first segmented images (SI₁) from the first segmentation unit (SU₁) in which the prostate gland, if present, is segmented,
∘ computing a first segmentation loss (Li), the first segmentation loss (L₁) quantifying deviations between the one or more first segmented images (SI₁) and the one or more target images (SI) of the segmented prostate gland,
∘ inputting the one or more first segmented images (SI₁) and the multi-parametric MRI image set (IS) of the patient into the second segmentation unit (SU₂),
∘ receiving one or more second segmented images (SI₂) from the second segmentation unit (SU₂) in which the prostatic and extra-prostatic cancer lesions, if present, are segmented,
∘ computing a second segmentation loss (L₂), the second segmentation loss (L₂) quantifying deviations between the one or more second segmented images (SI₂) and the one or more target images (SI) of the segmented prostatic and extra-prostatic cancer lesions,
∘ inputting the one or more second segmented images (SI₂) into the classification unit (CU),
∘ receiving the predicted prostate cancer local stage (PCS^{P}) from the classification unit (CU),
∘ computing the classification loss (L₃), the classification loss (L₃) quantifying deviations between the prostate cancer local stage (PCS) and the predicted prostate cancer local stage (PCS^{P}),
∘ modifying model parameters to reduce the first segmentation loss (L₃), the second segmentation loss (L₂), and the classification loss (L₃).

4. The method according to claim 2 or 3, wherein the multi-parametric MRI image set (IS, IS*) comprises one or more T2-weighted images and/or one or more apparent diffusion coefficient maps.

5. The method according to claim 2 or 3, wherein the multi-parametric MRI image set (IS, IS*) consists of one or more T2-weighted images and/or one or more apparent diffusion coefficient maps.

6. The method according to any one of claims 2 to 5, wherein each class corresponds to a prostate cancer local stage according to the tumor, nodes, and metastases staging system developed by the American Joint Committee on Cancer.

7. The method according to any one of claims 2 to 6, wherein each of the at least one segmentation unit (SU₁, SU₂) and classification unit (CU) is trained separately.

8. The method according to any one of claims 2 to 7, wherein each of the at least one segmentation unit (SU₁, SU₂) and classification unit (CU) is or comprises an artificial neural network.

9. A computer system (1) comprising:
a processor (20); and a memory (50) storing an application program (60) configured to perform, when executed by the processor (20), an operation, the operation comprising:
- receiving patient data, the patient data comprising a multi-parametric MRI image set (IS*) of an examination region comprising a prostate region of the patient,
- inputting the patient data into a trained machine learning model (MLM'), wherein the trained machine learning model (MLM^{t}) is configured and trained on training data (TD) to
o segment prostatic and extra-prostatic cancer lesions and generate one or more segmented images (SI₁*, SI₂*) based on the patient data,
o assign the one or more segmented images (SI₁*, SI₂*) to one of at least two classes, wherein each class corresponds to a prostate cancer local stage,
- receiving from the trained machine learning model (MLM') a predicted prostate cancer local stage (PCS^{P}*) and optionally the one or more segmented images (SI₁*, SI₂*) for the patient,
- outputting the predicted prostate cancer local stage (PCS^{P}*) and optionally the one or more segmented images (SI₁*, SI₂*), and/or storing the predicted prostate cancer local stage (PCS^{P}*) and optionally the one or more segmented images (SI₁*, SI₂*) on a data storage, and/or transmitting the predicted prostate cancer local stage (PCS^{P}*) and optionally the one or more segmented images (SI₁*, SI₂*) to a remote computer system,
wherein the machine learning model (MLM') comprises a first segmentation unit (SU₁) and a second segmentation unit (SU₂),
- wherein the first segmentation unit (SU₁) is configured and trained to receive the multi-parametric MRI image set (IS*) of the examination region and to generate one or more first segmented images (SI₁*) based on the multi-parametric MRI image set (IS*), wherein the prostate gland, if present is segmented in the one or more first segmented images (SI₁*),
- wherein the second segmentation unit (SU₂) is configured and trained to receive the one or more first segmented images (SI₁*) and the multi-parametric MRI image set (IS*) of the examination region and to generate one or more second segmented images (SI₂*) based on the first segmented images (SI₁*) and the multi-parametric MRI image set (IS*), wherein the prostatic cancer lesions and extra-prostatic cancer lesions, if present, are segmented in the one or more second segmented images (SI₂*),
the operation comprising:
- inputting the multi-parametric MRI image set (IS*) into the first segmentation unit (SU₁),
- receiving the one or more first segmented images (SI₁*) from the first segmentation unit (SU₁),
- inputting the multi-parametric MRI image set (IS*) and the first segmented images (SI₁*) into the second segmentation unit (SU₂),
- receiving the one or more second segmented images (SI₂*) from the second segmentation unit (SU₂),
- inputting the one or more second segmented images (SI₂*) into a classification unit (CU),
- receiving from the classification unit (CU) the predicted prostate cancer local stage (PCS^{P}*),
- outputting the predicted prostate cancer local stage (PCS^{P}*) and optionally the one or more first and/or second segmented images (SI₁*, SI₂*), and/or storing the predicted prostate cancer local stage (PCS^{P}*) and optionally the one or more first and/or second segmented images (SI₁*, SI₂*) on a data storage, and/or transmitting the predicted prostate cancer local stage (PCS^{P}*) and optionally the one or more first and/or second segmented images (SI₁*, SI₂*) to a remote computer system.

10. A non-transitory computer readable medium having stored thereon software instructions that, when executed by a processor (20) of a computer system (1), cause the computer system (1) to execute the following steps:
- receiving patient data, the patient data comprising a multi-parametric MRI image set (IS*) of an examination region comprising a prostate region of the patient,
- inputting the patient data into a trained machine learning model (MLM'), wherein the trained machine learning model (MLM^{t}) is configured and trained on training data (TD) to
∘ segment prostatic and extra-prostatic cancer lesions and generate one or more segmented images (SI₁*, SI₂*) based on the patient data,
∘ assign the one or more segmented images (SI₁*, SI₂*) to one of at least two classes, wherein each class corresponds to a prostate cancer local stage,
- receiving from the trained machine learning model (MLM') a predicted prostate cancer local stage (PCS^{P}*) and optionally the one or more segmented images (SI₁*, SI₂*) for the patient,
- outputting the predicted prostate cancer local stage (PCS^{P}*) and optionally the one or more segmented images (SI₁*, SI₂*), and/or storing the predicted prostate cancer local stage (PCS^{P}*) and optionally the one or more segmented images (SI₁*, SI₂*) on a data storage, and/or transmitting the predicted prostate cancer local stage (PCS^{P}*) and optionally the one or more segmented images (SI₁*, SI₂*) to a remote computer system,
wherein the machine learning model (MLM') comprises a first segmentation unit (SU₁) and a second segmentation unit (SU₂),
- wherein the first segmentation unit (SU₁) is configured and trained to receive the multi-parametric MRI image set (IS*) of the examination region and to generate one or more first segmented images (SI₁*) based on the multi-parametric MRI image set (IS*), wherein the prostate gland, if present is segmented in the one or more first segmented images (SI₁*),
- wherein the second segmentation unit (SU₂) is configured and trained to receive the one or more first segmented images (SI₁*) and the multi-parametric MRI image set (IS*) of the examination region and to generate one or more second segmented images (SI₂*) based on the first segmented images (SI₁*) and the multi-parametric MRI image set (IS*), wherein the prostatic cancer lesions and extra-prostatic cancer lesions, if present, are segmented in the one or more second segmented images (SI₂*),
wherein the software instructions cause the computer system (1) to execute the following steps:
- inputting the multi-parametric MRI image set (IS*) into the first segmentation unit (SU₁),
- receiving the one or more first segmented images (SI₁*) from the first segmentation unit (SU₁),
- inputting the multi-parametric MRI image set (IS*) and the first segmented images (SI₁*) into the second segmentation unit (SU₂),
- receiving the one or more second segmented images (SI₂*) from the second segmentation unit (SU₂),
- inputting the one or more second segmented images (SI₂*) into a classification unit (CU),
- receiving from the classification unit (CU) the predicted prostate cancer local stage (PCS^{P}*),
- outputting the predicted prostate cancer local stage (PCS^{P}*) and optionally the one or more first and/or second segmented images (SI₁*, SI₂*), and/or storing the predicted prostate cancer local stage (PCS^{P}*) and optionally the one or more first and/or second segmented images (SI₁*, SI₂*) on a data storage, and/or transmitting the predicted prostate cancer local stage (PCS^{P}*) and optionally the one or more first and/or second segmented images (SI₁*, SI₂*) to a remote computer system.

## Patentansprüche

1. Computerimplementiertes Verfahren zum Prognostizieren eines lokalen Stadiums von Prostatakrebs bei einem Patienten, wobei das Prognoseverfahren umfasst:
- Bereitstellen eines trainierten Maschinenlernmodells (MLM^{t}),
- Empfangen von Patientendaten, wobei die Patientendaten einen multiparametrischen MRI-Bilddatensatz (IS*) einer Untersuchungsregion umfassen, die eine Prostataregion des Patienten umfasst,
- Eingeben der Patientendaten in das trainierte Maschinenlernmodell (MLM^{t}), wobei das trainierte Maschinenlernmodell (MLM^{t}) anhand von Trainingsdaten ausgestaltet und trainiert wird zum
∘ Segmentieren prostatischer und extraprostatischer Krebsläsionen und Erzeugen eines oder mehrerer segmentierter Bilder (SI₁*, SI₂*) basierend auf den Patientendaten,
∘ Zuordnen der ein oder mehreren segmentierten Bilder (SI₁*, SI₂*) zu einer von mindestens zwei Klassen, wobei jede Klasse einem lokalen Stadium des Prostatakrebses entspricht,
- Empfangen, von dem trainierten Maschinenlernmodell (MLMt), eines prognostizierten lokalen Stadiums des Prostatakrebses (PCS^{P}*) und optional eines oder mehrerer segmentierter Bilder (SI₁*, SI₂*) für den Patienten,
- Ausgeben des prognostizierten lokalen Stadiums des Prostatakrebses (PCS^{P}*) und optional der ein oder mehreren segmentierten Bilder (SI₁*, SI₂*) und/oder Speichern des prognostizierten lokalen Stadiums des Prostatakrebses (PCS^{P}*) und optional der ein oder mehreren segmentierten Bilder (SI₁*, SI₂*) in einem Datenspeicher und/oder Übertragen des prognostizierten lokalen Stadiums des Prostatakrebses (PCS^{P}*) und optional der ein oder mehreren segmentierten Bilder (SI₁*, SI₂*) an ein entferntes Computersystem,
wobei das Maschinenlernmodell (MLM^{t}) eine erste Segmentierungseinheit (SU₁) und eine zweite Segmentierungseinheit (SU₂) umfasst,
- wobei die erste Segmentierungseinheit (SU₁) dafür ausgestaltet und trainiert ist, den multiparametrischen MRI-Bilddatensatz (IS*) der Untersuchungsregion zu empfangen und basierend auf dem multiparametrischen MRI-Bilddatensatz (IS*) die ein oder mehreren ersten segmentierten Bilder (SI₁*) zu erzeugen, wobei die Prostata, falls vorhanden, in den ein oder mehreren ersten segmentierten Bildern (SI₁*) segmentiert ist,
- wobei die zweite Segmentierungseinheit (SU₂) dafür ausgestaltet und trainiert ist, die ein oder mehreren ersten segmentierten Bilder (SI₁*) und den multiparametrischen MRI-Bilddatensatz (IS*) der Untersuchungsregion zu empfangen und die ein oder mehreren zweiten segmentierten Bilder (SI₂*) basierend auf den ersten segmentierten Bildern (SI₁*) und dem multiparametrischen MRI-Bilddatensatz (IS*) zu erzeugen, wobei die prostatischen Krebsläsionen und extraprostatischen Krebsläsionen, falls vorhanden, in den ein oder mehreren zweiten segmentierten Bildern (SI₂*) segmentiert sind,
wobei das Verfahren umfasst:
- Eingeben des multiparametrischen MRI-Bilddatensatzes (IS*) in die erste Segmentierungseinheit (SU₁),
- Empfangen der ein oder mehreren ersten segmentierten Bilder (SI₁*) von der ersten Segmentierungseinheit (SU₁),
- Eingeben des multiparametrischen MRI-Bilddatensatzes (IS*) und der ersten segmentierten Bilder (SI₁*) in die zweite Segmentierungseinheit (SU₂),
- Empfangen der ein oder mehreren zweiten segmentierten Bilder (SI₂*) von der zweiten Segmentierungseinheit (SU₂),
- Eingeben der ein oder mehreren zweiten segmentierten Bilder (SI₂*) in eine Klassifizierungseinheit (CU),
- Empfangen, von der Klassifizierungseinheit (CU), des prognostizierten lokalen Stadiums des Prostatakrebses (PCS^{P}*),
- Ausgeben des prognostizierten lokalen Stadiums des Prostatakrebses (PCS^{P}*) und optional der ein oder mehreren ersten und/oder zweiten segmentierten Bilder (SI₁*, SI₂*) und/oder Speichern des prognostizierten lokalen Stadiums des Prostatakrebses (PCS^{P}*) und optional der ein oder mehreren ersten und/oder zweiten segmentierten Bilder (SI₁*, SI₂*) in einem Datenspeicher und/oder Übertragen des prognostizierten lokalen Stadiums des Prostatakrebses (PCS^{P}*) und optional der ein oder mehreren ersten und/oder zweiten segmentierten Bilder (SI₁*, SI₂*) an ein entferntes Computersystem.

2. Verfahren gemäß Anspruch 1, wobei das trainierte Maschinenlernmodell (MLM^{t}) nach einem Trainingsverfahren trainiert wurde, wobei das Trainingsverfahren umfasst:
- Empfangen und/oder Bereitstellen eines Maschinenlernmodells (MLM), wobei das Maschinenlernmodell (MLM) mindestens eine Segmentierungseinheit (SU₁. SU₂) und eine Klassifizierungseinheit (CU) umfasst, wobei die mindestens eine Segmentierungseinheit (SU₁. SU₂) dafür ausgestaltet ist, einen multiparametrischen MRI-Bilddatensatz (IS) einer Untersuchungsregion, die eine Prostataregion eines männlichen Menschen umfasst, zu empfangen und ein oder mehrere segmentierte Bilder (SI₁, SI₂) basierend auf den ein oder mehreren empfangenen Bildern und Modellparametern zu erzeugen, und wobei die Klassifizierungseinheit (CU) dafür ausgestaltet ist, die ein oder mehreren segmentierten Bilder (SI₁, SI₂) basierend auf Modellparametern einer von mindestens zwei Klassen zuzuordnen, wobei jede Klasse einem lokalen Stadium des Prostatakrebses entspricht,
- Empfangen und/oder Bereitstellen von Trainingsdaten (TD), wobei die Trainingsdaten (TD) umfassen, für jeden Patienten einer Vielzahl von Patienten: Eingabedaten und Zieldaten, wobei die Eingabedaten einen multiparametrischen MRI-Bilddatensatz (IS) einer Untersuchungsregion, die eine Prostataregion des Patienten umfasst, umfassen und die Zieldaten ein oder mehrere Zielbilder (SI), in denen, falls vorhanden, Prostata, prostatische Krebsläsionen und extraprostatische Krebsläsionen segmentiert sind, und ein lokales Stadium des Prostatakrebses (PCS) für den Patienten umfassen,
- Trainieren des Maschinenlernmodells (MLM), wobei das Trainieren für jeden Patienten aus der Vielzahl von Patienten umfasst:
∘ Eingeben des multiparametrischen MRI-Bilddatensatzes (IS) des Patienten in mindestens eine Segmentierungseinheit (SU₁. SU₂),
∘ Empfangen eines oder mehrerer segmentierter Bilder (SI₁. SI₂) von der mindestens einen Segmentierungseinheit (SU₁. SU₂), in denen, falls vorhanden, prostatische und extraprostatische Krebsläsionen segmentiert sind,
∘ Berechnen eines Segmentierungsverlusts (L₁, L₂), wobei der Segmentierungsverlust (L₁, L₂) die Abweichungen zwischen den ein oder mehreren segmentierten Bildern (SI₁. SI₂) und den ein oder mehreren Zielbildern (SI) quantifiziert,
o Eingeben der ein oder mehreren segmentierten Bilder (SI₁. SI₂) in die Klassifizierungseinheit (CU),
o Empfangen eines prognostizierten lokalen Stadiums des Prostatakrebses (PCS^{P}) von der Klassifizierungseinheit (CU),
o Berechnen eines Klassifizierungsverlusts (L₃), wobei der Klassifizierungsverlust (L₃) die Abweichungen zwischen dem lokalen Stadium des Prostatakrebses (PCS) und dem vorhergesagten lokalen Stadium des Prostatakrebses (PCS^{P}) quantifiziert,
o Modifizieren von Modellparametern zum Reduzieren des Segmentierungsverlusts (L₁, L₂) und des Klassifizierungsverlusts (L₃),
- Speichern und/oder Ausgeben der Modellparameter und/oder des trainierten Maschinenlernmodells (MLM^{t}) und/oder Übertragen der Modellparameter und/oder des trainierten Maschinenlernmodells (MLM^{t}) an ein entferntes Computersystem und/oder Verwenden des trainierten Maschinenlernmodells (MLM^{t}) zum Vorhersagen eines lokalen Stadiums des Prostatakrebses für einen neuen Patienten.

3. Verfahren gemäß Anspruch 2,
- wobei das Trainieren für jeden Patienten aus der Vielzahl von Patienten umfasst:
∘ Eingeben des multiparametrischen MRI-Bilddatensatzes (IS) des Patienten in die erste Segmentierungseinheit (SU₁),
∘ Empfangen eines oder mehrerer erster segmentierter Bilder (SI₁) von der ersten Segmentierungseinheit (SU₁), in denen die Prostata, falls vorhanden, segmentiert ist,
∘ Berechnen eines ersten Segmentierungsverlusts (L₁), wobei der erste Segmentierungsverlust (L₁) die Abweichungen zwischen den ein oder mehreren ersten segmentierten Bildern (SI₁) und den ein oder mehreren Zielbildern (SI) der segmentierten Prostata quantifiziert,
o Eingeben der ein oder mehreren ersten segmentierten Bilder (SI₁) und des multiparametrischen MRI-Bilddatensatzes (IS) des Patienten in die zweite Segmentierungseinheit (SU₂),
o Empfangen eines oder mehrerer zweiter segmentierter Bilder (SI₂) von der zweiten Segmentierungseinheit (SU₂), in denen die prostatischen und extraprostatischen Krebsläsionen, falls vorhanden, segmentiert sind,
o Berechnen eines zweiten Segmentierungsverlusts (L₂), wobei der zweite Segmentierungsverlust (L₂) Abweichungen zwischen den ein oder mehreren zweiten segmentierten Bildern (SI₂) und den ein oder mehreren Zielbildern (SI) der segmentierten prostatischen und extraprostatischen Krebsläsionen quantifiziert,
o Eingeben der ein oder mehreren zweiten segmentierten Bilder (SI₂) in die Klassifizierungseinheit (CU),
o Empfangen des prognostizierten lokalen Stadiums des Prostatakrebses (PCS^{P}) von der Klassifizierungseinheit (CU),
o Berechnen des Klassifizierungsverlusts (L₃), wobei der Klassifizierungsverlust (L₃) Abweichungen zwischen dem lokalen Stadium des Prostatakrebses (PCS) und dem vorhergesagten lokalen Stadium des Prostatakrebses (PCS^{P}) quantifiziert,
o Modifizieren von Modellparametern zum Reduzieren des ersten Segmentierungsverlusts (L₃), des zweiten Segmentierungsverlusts (L₂) und des Klassifizierungsverlusts (L₃).

4. Verfahren gemäß Anspruch 2 oder 3, wobei der multiparametrische MRI-Bilddatensatz (IS, IS*) ein oder mehrere T2-gewichtete Bilder und/oder eine oder mehrere Karten der scheinbaren Diffusionskoeffizienten umfasst.

5. Verfahren gemäß Anspruch 2 oder 3, wobei der multiparametrische MRI-Bilddatensatz (IS, IS*) aus ein oder mehreren T2-gewichteten Bildern und/oder einer oder mehreren Karten der scheinbaren Diffusionskoeffizienten besteht.

6. Verfahren gemäß einem der Ansprüche 2 bis 5, wobei jede Klasse einem lokalen Stadium des Prostatakrebses gemäß dem vom American Joint Committee on Cancer entwickelten Tumor-, Lymphknoten- und Metastasen-Stadienbestimmungssystem entspricht.

7. Verfahren gemäß einem der Ansprüche 2 bis 6, wobei jede der mindestens einen Segmentierungseinheit (SU₁, SU₂) und Klassifizierungseinheit (CU) separat trainiert wird.

8. Verfahren gemäß einem der Ansprüche 2 bis 7, wobei jede der mindestens einen Segmentierungseinheit (SU₁, SU₂) und Klassifizierungseinheit (CU) ein künstliches neuronales Netz ist oder umfasst.

9. Computersystem (1), umfassend:
einen Prozessor (20); und einen Speicher (50), der ein Anwendungsprogramm (60) speichert, das dafür ausgestaltet ist, bei Ausführung durch den Prozessor (20) einen Vorgang auszuführen, wobei der Vorgang umfasst:
- Empfangen von Patientendaten, wobei die Patientendaten einen multiparametrischen MRI-Bilddatensatz (IS*) einer Untersuchungsregion umfassen, die eine Prostataregion des Patienten umfasst,
- Eingeben der Patientendaten in ein trainiertes Maschinenlernmodell (MLM^{t}), wobei das trainierte Maschinenlernmodell (MLM^{t}) anhand von Trainingsdaten (TD) ausgestaltet und trainiert wird zum
∘ Segmentieren prostatischer und extraprostatischer Krebsläsionen und Erzeugen eines oder mehrerer segmentierter Bilder (SI₁*, SI₂*) basierend auf den Patientendaten,
o Zuordnen der ein oder mehreren segmentierten Bilder (SI₁*, SI₂*) zu einer von mindestens zwei Klassen, wobei jede Klasse einem lokalen Stadium des Prostatakrebses entspricht,
- Empfangen, von dem trainierten Maschinenlernmodell (MLMt), eines prognostizierten lokalen Stadiums des Prostatakrebses (PCS^{P}*) und optional der ein oder mehreren segmentierten Bilder (SI₁*, SI₂*) für den Patienten,
- Ausgeben des prognostizierten lokalen Stadiums des Prostatakrebses (PCS^{P}*) und optional der ein oder mehreren segmentierten Bilder (SI₁*, SI₂*) und/oder Speichern des prognostizierten lokalen Stadiums des Prostatakrebses (PCS^{P}*) und optional der ein oder mehreren segmentierten Bilder (SI₁*, SI₂*) in einem Datenspeicher und/oder Übertragen des prognostizierten lokalen Stadiums des Prostatakrebses (PCS^{P}*) und optional der ein oder mehreren segmentierten Bilder (SI₁*, SI₂*) an ein entferntes Computersystem,
wobei das Maschinenlernmodell (MLM^{t}) eine erste Segmentierungseinheit (SU₁) und eine zweite Segmentierungseinheit (SU₂) umfasst,
- wobei die erste Segmentierungseinheit (SU₁) dafür ausgestaltet und trainiert ist, den multiparametrischen MRI-Bilddatensatz (IS*) der Untersuchungsregion zu empfangen und basierend auf dem multiparametrischen MRI-Bilddatensatz (IS*) ein oder mehrere erste segmentierte Bilder (SI₁*) zu erzeugen, wobei die Prostata, falls vorhanden, in den ein oder mehreren ersten segmentierten Bildern (SI₁*) segmentiert ist,
- wobei die zweite Segmentierungseinheit (SU₂) dafür ausgestaltet und trainiert ist, die ein oder mehreren ersten segmentierten Bilder (SI₁*) und den multiparametrischen MRI-Bilddatensatz (IS*) der Untersuchungsregion zu empfangen und ein oder mehrere zweite segmentierte Bilder (SI₂*) basierend auf den ersten segmentierten Bildern (SI₁*) und dem multiparametrischen MRI-Bilddatensatz (IS*) zu erzeugen, wobei die prostatischen Krebsläsionen und extraprostatischen Krebsläsionen, falls vorhanden, in den ein oder mehreren zweiten segmentierten Bildern (SI₂*) segmentiert sind,
wobei der Vorgang umfasst:
- Eingeben des multiparametrischen MRI-Bilddatensatzes (IS*) in die erste Segmentierungseinheit (SU₁),
- Empfangen der ein oder mehreren ersten segmentierten Bilder (SI₁*) von der ersten Segmentierungseinheit (SU₁),
- Eingeben des multiparametrischen MRI-Bilddatensatzes (IS*) und der ersten segmentierten Bilder (SI₁*) in die zweite Segmentierungseinheit (SU₂),
- Empfangen der ein oder mehreren zweiten segmentierten Bilder (SI₂*) von der zweiten Segmentierungseinheit (SU₂),
- Eingeben der ein oder mehreren zweiten segmentierten Bilder (SI₂*) in eine Klassifizierungseinheit (CU),
- Empfangen, von der Klassifizierungseinheit (CU), des prognostizierten lokalen Stadiums des Prostatakrebses (PCS^{P}*),
- Ausgeben des prognostizierten lokalen Stadiums des Prostatakrebses (PCS^{P}*) und optional der ein oder mehreren ersten und/oder zweiten segmentierten Bilder (SI₁*, SI₂*) und/oder Speichern des prognostizierten lokalen Stadiums des Prostatakrebses (PCS^{P}*) und optional der ein oder mehreren ersten und/oder zweiten segmentierten Bilder (SI₁*, SI₂*) in einem Datenspeicher und/oder Übertragen des prognostizierten lokalen Stadiums des Prostatakrebses (PCS^{P}*) und optional der ein oder mehreren ersten und/oder zweiten segmentierten Bilder (SI₁*, SI₂*) an ein entferntes Computersystem,

10. Nichtflüchtiges, computerlesbares Medium, auf dem Software-Anweisungen gespeichert sind, die, wenn sie von einem Prozessor (20) eines Computersystems (1) ausgeführt werden, das Computersystem (1) veranlassen, die folgenden Schritte auszuführen:
- Empfangen von Patientendaten, wobei die Patientendaten einen multiparametrischen MRI-Bilddatensatz (IS*) einer Untersuchungsregion umfassen, die eine Prostataregion des Patienten umfasst,
- Eingeben der Patientendaten in ein trainiertes Maschinenlernmodell (MLM^{t}), wobei das trainierte Maschinenlernmodell (MLM^{t}) anhand von Trainingsdaten (TD) ausgestaltet und trainiert wird zum
∘ Segmentieren prostatischer und extraprostatischer Krebsläsionen und Erzeugen eines oder mehrerer segmentierter Bilder (SI₁*, SI₂*) basierend auf den Patientendaten,
∘ Zuordnen der ein oder mehreren segmentierten Bilder (SI₁*, SI₂*) zu einer von mindestens zwei Klassen, wobei jede Klasse einem lokalen Stadium des Prostatakrebses entspricht,
- Empfangen, von dem trainierten Maschinenlernmodell (MLM^{t}), eines prognostizierten lokalen Stadiums des Prostatakrebses (PCS^{P}*) und optional der ein oder mehreren segmentierten Bilder (SI₁*, SI₂*) für den Patienten,
- Ausgeben des prognostizierten lokalen Stadiums des Prostatakrebses (PCS^{P}*) und optional der ein oder mehreren segmentierten Bilder (SI₁*, SI₂*) und/oder Speichern des prognostizierten lokalen Stadiums des Prostatakrebses (PCS^{P}*) und optional der ein oder mehreren segmentierten Bilder (SI₁*, SI₂*) in einem Datenspeicher und/oder Übertragen des prognostizierten lokalen Stadiums des Prostatakrebses (PCS^{P}*) und optional der ein oder mehreren segmentierten Bilder (SI₁*, SI₂*) an ein entferntes Computersystem,
wobei das Maschinenlernmodell (MLM^{t}) eine erste Segmentierungseinheit (SU₁) und eine zweite Segmentierungseinheit (SU₂) umfasst,
- wobei die erste Segmentierungseinheit (SU₁) dafür ausgestaltet und trainiert ist, den multiparametrischen MRI-Bilddatensatz (IS*) der Untersuchungsregion zu empfangen und basierend auf dem multiparametrischen MRI-Bilddatensatz (IS*) ein oder mehrere erste segmentierte Bilder (SI₁*) zu erzeugen, wobei die Prostata, falls vorhanden, in den ein oder mehreren ersten segmentierten Bildern (SI₁*) segmentiert ist,
- wobei die zweite Segmentierungseinheit (SU₂) dafür ausgestaltet und trainiert ist, die ein oder mehreren ersten segmentierten Bilder (SI₁*) und den multiparametrischen MRI-Bilddatensatz (IS*) der Untersuchungsregion zu empfangen und ein oder mehrere zweite segmentierte Bilder (SI₂*) basierend auf den ersten segmentierten Bildern (SI₁*) und dem multiparametrischen MRI-Bilddatensatz (IS*) zu erzeugen, wobei die prostatischen Krebsläsionen und extraprostatischen Krebsläsionen, falls vorhanden, in den ein oder mehreren zweiten segmentierten Bildern (SI₂*) segmentiert sind,
wobei die Softwareanweisungen das Computersystem (1) veranlassen, die folgenden Schritte auszuführen:
- Eingeben des multiparametrischen MRI-Bilddatensatzes (IS*) in die erste Segmentierungseinheit (SU₁),
- Empfangen der ein oder mehreren ersten segmentierten Bilder (SI₁*) von der ersten Segmentierungseinheit (SU₁),
- Eingeben des multiparametrischen MRI-Bilddatensatzes (IS*) und der ersten segmentierten Bilder (SI₁*) in die zweite Segmentierungseinheit (SU₂),
- Empfangen der ein oder mehreren zweiten segmentierten Bilder (SI₂*) von der zweiten Segmentierungseinheit (SU₂),
- Eingeben der ein oder mehreren zweiten segmentierten Bilder (SI₂*) in eine Klassifizierungseinheit (CU),
- Empfangen, von der Klassifizierungseinheit (CU), des prognostizierten lokalen Stadiums des Prostatakrebses (PCS^{P}*),
- Ausgeben des prognostizierten lokalen Stadiums des Prostatakrebses (PCS^{P}*) und optional der ein oder mehreren ersten und/oder zweiten segmentierten Bilder (SI₁*, SI₂*) und/oder Speichern des prognostizierten lokalen Stadiums des Prostatakrebses (PCS^{P}*) und optional der ein oder mehreren ersten und/oder zweiten segmentierten Bilder (SI₁*, SI₂*) in einem Datenspeicher und/oder Übertragen des prognostizierten lokalen Stadiums des Prostatakrebses (PCS^{P}*) und optional der ein oder mehreren ersten und/oder zweiten segmentierten Bilder (SI₁*, SI₂*) an ein entferntes Computersystem.

## Revendications

1. Procédé mis en œuvre par ordinateur pour prédire un stade local de cancer de la prostate pour un patient, le procédé de prédiction comprenant :
- la fourniture d'un modèle d'apprentissage automatique entraîné (MLM^{t}),
- la réception de données du patient, les données du patient comprenant un ensemble d'images IRM multiparamétriques (IS*) d'une région d'examen comprenant une région de la prostate du patient,
- l'entrée des données du patient dans le modèle d'apprentissage automatique entraîné (MLM^{t}), le modèle d'apprentissage automatique entraîné (MLM^{t}) étant configuré et entraîné sur des données d'entraînement pour
∘ segmenter des lésions cancéreuses prostatiques et extra-prostatiques et générer une ou plusieurs images segmentées (SI₁*, SI₂*) sur la base des données du patient,
∘ affecter la ou les images segmentées (SI₁*, SI₂*) à une d'au moins deux classes , chaque classe correspondant à un stade local du cancer de la prostate,
- la réception en provenance du modèle d'apprentissage automatique entraîné (MLM^{t}) d'un stade local prédit du cancer de la prostate (PCS^{P}*) et éventuellement de la ou des images segmentées (SI₁*, SI₂*) pour le patient,
- l'émission du stade local prédit du cancer de la prostate (PCS^{P}*) et éventuellement de la ou des images segmentées (SI₁*, SI₂*), et/ou le stockage du stade local prédit du cancer de la prostate (PCS^{P}*) et éventuellement de la ou des images segmentées (SI₁*, SI₂*) sur un support de données, et/ou la transmission du stade local prédit du cancer de la prostate (PCS^{P}*) et éventuellement de la ou des images segmentées (SI₁*, SI₂*) à un système informatique à distance,
le modèle d'apprentissage automatique (MLM^{t}) comprenant une première unité de segmentation (SU₁) et une seconde unité de segmentation (SU₂),
- la première unité de segmentation (SU₁) étant configurée et entraînée pour recevoir l'ensemble d'images IRM multiparamétriques (IS*) de la région d'examen et pour générer la ou les premières images segmentées (SI₁*) sur la base de l'ensemble d'images IRM multiparamétriques (IS*), la prostate, si elle est présente, étant segmentée dans la ou les premières images segmentées (SI₁*),
- la seconde unité de segmentation (SU₂) étant configurée et entraînée pour recevoir la ou les premières images segmentées (SI₁*) et l'ensemble d'images IRM multiparamétriques (IS*) de la région d'examen et pour générer la ou les secondes images segmentées (SI₂*) sur la base des premières images segmentées (SI₁*) et de l'ensemble d'images IRM multiparamétriques (IS*), les lésions cancéreuses prostatiques et les lésions cancéreuses extra-prostatiques, si elles sont présentes, étant segmentées dans la ou les secondes images segmentées (SI₂*),
le procédé comprenant :
- l'entrée de l'ensemble d'images IRM multiparamétriques (IS*) dans la première unité de segmentation (SU₁),
- la réception de la ou des premières images segmentées (SI₁*) en provenance de la première unité de segmentation (SU₁),
- l'entrée de l'ensemble d'images IRM multiparamétriques (IS*) et des premières images segmentées (SI₁*) dans la seconde unité de segmentation (SU₂),
- la réception de la ou des secondes images segmentées (SI₂*) en provenance de la seconde unité de segmentation (SU₂),
- l'entrée de la ou des secondes images segmentées (SI₂*) dans une unité de classification (CU),
- la réception en provenance de l'unité de classification (CU) du stade local prédit du cancer de la prostate (PCS^{P}*),
- l'émission du stade local prédit du cancer de la prostate (PCS^{P}*) et éventuellement de la ou des premières et/ou secondes images segmentées (SI₁*, SI₂*), et/ou le stockage du stade local prédit du cancer de la prostate (PCS^{P}*) et éventuellement de la ou des premières et/ou secondes images segmentées (SI₁*, SI₂*) sur une mémoire de données, et/ou la transmission du stade local prédit du cancer de la prostate (PCS^{P}*) et éventuellement de la ou des premières et/ou secondes images segmentées (SI₁*, SI₂*) à un système informatique à distance.

2. Procédé selon la revendication 1, le modèle d'apprentissage automatique (MLM^{t}) entraîné ayant été entraîné dans un procédé d'entraînement, le procédé d'entraînement comprenant :
- la réception et/ou la fourniture d'un modèle d'apprentissage automatique (MLM), le modèle d'apprentissage automatique (MLM) comprenant au moins une unité de segmentation (SU₁. SU₂) et une unité de classification (CU), l'au moins une unité de segmentation étant (SU₁. SU₂) configurée pour recevoir un ensemble d'images IRM multiparamétriques (IS) d'une région d'examen comprenant une région de la prostate d'un homme et pour générer une ou plusieurs images segmentées (SI₁, SI₂) sur la base de la ou des images reçues et des paramètres du modèle, et l'unité de classification (CU) étant configurée pour affecter la ou les images segmentées (SI₁, SI₂) à l'une d'au moins deux classes sur la base des paramètres du modèle, chaque classe correspondant à un stade local du cancer de la prostate,
- la réception et/ou la fourniture de données d'entraînement (TD), les données d'entraînement (TD) comprenant, pour chaque patient d'une multitude de patients, des données d'entrée et des données cibles, les données d'entrée comprenant un ensemble d'images IRM multiparamétriques (IS) d'une région d'examen comprenant une région de la prostate du patient, et les données cibles comprenant une ou plusieurs images cibles (SI) dans lesquelles, si elles sont présentes, la glande prostatique, les lésions cancéreuses prostatiques et les lésions cancéreuses extra-prostatiques sont segmentées, et un stade local du cancer de la prostate (PCS) pour le patient,
- l'entraînement du modèle d'apprentissage automatique (MLM), l'entraînement comprenant pour chaque patient de la multitude de patients :
∘ l'entrée de l'ensemble d'images IRM multiparamétriques (IS) du patient dans l'au moins une unité de segmentation (SU₁. SU₂),
∘ la réception d'une ou plusieurs images segmentées (SI₁. SI₂) en provenance de l'au moins une unité de segmentation (SU₁. SU₂) dans laquelle, si elles sont présentes, les lésions cancéreuses prostatiques et extra-prostatiques sont segmentées,
∘ le calcul d'une perte de segmentation (L₁, L₂), la perte de segmentation (L₁, L₂) quantifiant des écarts entre la ou les images segmentées (SI₁. SI₂) et la ou les images cibles (SI),
∘ l'entrée de la ou des images segmentées (SI₁. SI₂) dans l'unité de classification (CU),
∘ la réception d'un stade local prédit du cancer de la prostate (PCS^{P}) en provenance de l'unité de classification (CU),
∘ le calcul d'une perte de classification (L₃), la perte de classification (L₃) quantifiant des écarts entre le stade local du cancer de la prostate (PCS) et le stade local prédit du cancer de la prostate (PCS^{P}),
∘ la modification de paramètres du modèle pour réduire la perte de segmentation (L₁, L₂) et la perte de classification (L₃),
- le stockage et/ou l'émission des paramètres du modèle et/ou du modèle d'apprentissage automatique entraîné (MLM^{t}) et/ou la transmission des paramètres du modèle et/ou du modèle d'apprentissage automatique entraîné (MLM^{t}) à un système informatique distant et/ou l'utilisation du modèle d'apprentissage automatique entraîné (MLM^{t}) pour prédire un stade local du cancer de la prostate d'un nouveau patient.

3. Procédé selon la revendication 2,
- l'entraînement comprenant pour chaque patient de la multitude de patients :
∘ l'entrée de l'ensemble d'images IRM multiparamétriques (IS) du patient dans la première unité de segmentation (SU₁),
∘ la réception d'une ou plusieurs premières images segmentées (SI₁) en provenance de la première unité de segmentation (SU₁) dans lesquelles la prostate, si elle est présente, est segmentée,
∘ le calcul d'une première perte de segmentation (L₁), la première perte de segmentation (L₁) quantifiant des écarts entre la ou les premières images segmentées (SI₁) et la ou les images cibles (SI) de la glande prostatique segmentée,
∘ l'entrée de la ou des premières images segmentées (SI₁) et de l'ensemble d'images IRM multiparamétriques (IS) du patient dans la seconde unité de segmentation (SU₂),
∘ la réception d'une ou plusieurs secondes images segmentées (SI₂) en provenance de la seconde unité de segmentation (SU₂) dans lesquelles les lésions cancéreuses prostatiques et extra-prostatiques, si elles sont présentes, sont segmentées,
∘ le calcul d'une seconde perte de segmentation (L₂), la seconde perte de segmentation (L₂) quantifiant des écarts entre la ou les secondes images segmentées (SI₂) et la ou les images cibles (SI) des lésions cancéreuses prostatiques et extra-prostatiques segmentées,
∘ l'entrée de la ou des secondes images segmentées (SI₂) dans l'unité de classification (CU),
∘ la réception du stade local prédit du cancer de la prostate (PCS^{P}) en provenance de l'unité de classification (CU),
∘ le calcul de la perte de classification (L₃), la perte de classification (L₃) quantifiant des écarts entre le stade local du cancer de la prostate (PCS) et le stade local prédit du cancer de la prostate (PCS^{P}),
∘ la modification de paramètres du modèle pour réduire la première perte de segmentation (L₃), la seconde perte de segmentation (L₂) et la perte de classification (L₃).

4. Procédé selon la revendication 2 ou 3, l'ensemble d'images IRM multiparamétriques (IS, IS*) comprenant une ou plusieurs images pondérées en T2 et/ou une ou plusieurs cartes de coefficient de diffusion apparent.

5. Procédé selon la revendication 2 ou 3, l'ensemble d'images IRM multiparamétriques (IS, IS*) comprenant une ou plusieurs images pondérées en T2 et/ou une ou plusieurs cartes de coefficient de diffusion apparent.

6. Procédé selon l'une quelconque des revendications 2 à 5, chaque classe correspondant à un stade local du cancer de la prostate selon le système de stadification des tumeurs, des ganglions et des métastases développé par l'American Joint Committee on Cancer.

7. Procédé selon l'une quelconque des revendications 2 à 6, chacune de l'au moins une unité de segmentation (SU₁, SU₂) et unité de classification (CU) étant entraînée séparément.

8. Procédé selon l'une quelconque des revendications 2 à 7, chacune de l'au moins une unité de segmentation (SU₁, SU₂) et unité de classification (CU) étant ou comprenant un réseau de neurones artificiels.

9. Système informatique (1) comprenant :
un processeur (20) ; et une mémoire (50) stockant un programme d'application (60) configuré pour effectuer, lorsqu'il est exécuté par le processeur (20), une opération, l'opération comprenant :
- la réception de données du patient, les données du patient comprenant un ensemble d'images IRM multiparamétriques (IS*) d'une région d'examen comprenant une région de la prostate du patient,
- l'entrée des données du patient dans un modèle d'apprentissage automatique entraîné (MLM^{t}), le modèle d'apprentissage automatique entraîné (MLM^{t}) étant configuré et entraîné sur des données d'entraînement (TD) pour
*∘* segmenter des lésions cancéreuses prostatiques et extra-prostatiques et générer une ou plusieurs images segmentées (SI₁*, SI₂*) sur la base des données du patient,
*∘* affecter la ou les images segmentées (SI₁*, SI₂*) à une d'au moins deux classes, chaque classe correspondant à un stade local du cancer de la prostate,
- la réception en provenance du modèle d'apprentissage automatique entraîné (MLMt) d'un stade local prédit du cancer de la prostate (PCS^{P}*) et éventuellement de la ou des images segmentées (SI₁*, SI₂*) pour le patient,
- l'émission du stade local prédit du cancer de la prostate (PCSP*) et éventuellement de la ou des images segmentées (SI₁*, SI₂*), et/ou le stockage du stade local prédit du cancer de la prostate (PCSP*) et éventuellement de la ou des images segmentées (SI₁*, SI₂*) sur un support de données, et/ou la transmission du stade local prédit du cancer de la prostate (PCSP*) et éventuellement de la ou des images segmentées (SI₁*, SI₂*) à un système informatique à distance,
le modèle d'apprentissage automatique (MLMt) comprenant une première unité de segmentation (SU₁) et une seconde unité de segmentation (SU₂),
- la première unité de segmentation (SU₁) étant configurée et entraînée pour recevoir l'ensemble d'images IRM multiparamétriques (IS*) de la région d'examen et pour générer une ou plusieurs premières images segmentées (SI₁*) sur la base de l'ensemble d'images IRM multiparamétriques (IS*), la prostate, si elle est présente, étant segmentée dans la ou les premières images segmentées (SI₁*),
- la seconde unité de segmentation (SU₂) étant configurée et entraînée pour recevoir la ou les premières images segmentées (SI₁*) et l'ensemble d'images IRM multiparamétriques (IS*) de la région d'examen et pour générer une ou plusieurs secondes images segmentées (SI₂*) sur la base des premières images segmentées (SI₁*) et de l'ensemble d'images IRM multiparamétriques (IS*), les lésions cancéreuses prostatiques et les lésions cancéreuses extra-prostatiques, si elles sont présentes, étant segmentées dans la ou les secondes images segmentées (SI₂*),
l'opération comprenant :
- l'entrée de l'ensemble d'images IRM multiparamétriques (IS*) dans la première unité de segmentation (SU₁),
- la réception de la ou des premières images segmentées (SI1*) en provenance de la première unité de segmentation (SU₁),
- l'entrée de l'ensemble d'images IRM multiparamétriques (IS*) et des premières images segmentées (SI1*) dans la seconde unité de segmentation (SU2),
- la réception de la ou des secondes images segmentées (SI₂*) en provenance de la seconde unité de segmentation (SU2),
- l'entrée de la ou des secondes images segmentées (SI₂*) dans une unité de classification (CU),
- la réception en provenance de l'unité de classification (CU) du stade local prédit du cancer de la prostate (PCS^{P}*),
- l'émission du stade local prédit du cancer de la prostate (PCSP*) et éventuellement de la ou des premières et/ou secondes images segmentées (SI₁*, SI₂*), et/ou le stockage du stade local prédit du cancer de la prostate (PCSP*) et éventuellement de la ou des premières et/ou secondes images segmentées (SI₁*, SI₂*) sur une mémoire de données, et/ou la transmission du stade local prédit du cancer de la prostate (PCSP*) et éventuellement de la ou des premières et/ou secondes images segmentées (SI₁*, SI₂*) à un système informatique à distance.

10. Support informatique non transitoire sur lequel sont stockées des instructions logicielles qui, lorsqu'elles sont exécutées par un processeur (20) d'un système informatique (1), amènent le système informatique (1) à exécuter les étapes suivantes :
- la réception de données du patient, les données du patient comprenant un ensemble d'images IRM multiparamétriques (IS*) d'une région d'examen comprenant une région de la prostate du patient,
- l'entrée des données du patient dans le modèle d'apprentissage automatique entraîné (MLM^{t}), le modèle d'apprentissage automatique entraîné (MLM^{t}) étant configuré et entraîné sur des données d'entraînement (TD) pour
*∘* segmenter des lésions cancéreuses prostatiques et extra-prostatiques et générer une ou plusieurs images segmentées (SI1*, SI₂*) sur la base des données du patient,
*∘* affecter la ou les images segmentées (SI₁*, SI₂*) à une d'au moins deux classes, chaque classe correspondant à un stade local du cancer de la prostate,
- la réception en provenance du modèle d'apprentissage automatique entraîné (MLMt) d'un stade local prédit du cancer de la prostate (PCS^{P}*) et éventuellement de la ou des images segmentées (SI1*, SI₂*) pour le patient,
- l'émission du stade local prédit du cancer de la prostate (PCSP*) et éventuellement de la ou des images segmentées (SI1*, SI₂*), et/ou le stockage du stade local prédit du cancer de la prostate (PCSP*) et éventuellement de la ou des images segmentées (SI1*, SI₂*) sur un support de données, et/ou la transmission du stade local prédit du cancer de la prostate (PCSP*) et éventuellement de la ou des images segmentées (SI1*, SI₂*) à un système informatique à distance,
le modèle d'apprentissage automatique (MLMt) comprenant une première unité de segmentation (SU₁) et une seconde unité de segmentation (SU₂),
- la première unité de segmentation (SU1) étant configurée et entraînée pour recevoir l'ensemble d'images IRM multiparamétriques (IS*) de la région d'examen et pour générer une ou plusieurs premières images segmentées (SI₁*) sur la base de l'ensemble d'images IRM multiparamétriques (IS*), la prostate, si elle est présente, étant segmentée dans la ou les premières images segmentées (SI₁*),
- la seconde unité de segmentation (SU₂) étant configurée et entraînée pour recevoir la ou les premières images segmentées (SI₁*) et l'ensemble d'images IRM multiparamétriques (IS*) de la région d'examen et pour générer une ou plusieurs secondes images segmentées (SI₂*) sur la base des premières images segmentées (SI₁*) et de l'ensemble d'images IRM multiparamétriques (IS*), les lésions cancéreuses prostatiques et les lésions cancéreuses extra-prostatiques, si elles sont présentes, étant segmentées dans la ou les secondes images segmentées (SI₂*),
les instructions logicielles amenant le système informatique (1) à exécuter les étapes suivantes :
- l'entrée de l'ensemble d'images IRM multiparamétriques (IS*) dans la première unité de segmentation (SU₁),
- la réception de la ou des premières images segmentées (SI1*) en provenance de la première unité de segmentation (SU1),
- l'entrée de l'ensemble d'images IRM multiparamétriques (IS*) et des premières images segmentées (SI1*) dans la seconde unité de segmentation (SU2),
- la réception de la ou des secondes images segmentées (SI₂*) en provenance de la seconde unité de segmentation (SU2),
- l'entrée de la ou des secondes images segmentées (SI₂*) dans une unité de classification (CU),
- la réception en provenance de l'unité de classification (CU) du stade local prédit du cancer de la prostate (PCS^{P}*),
- l'émission du stade local prédit du cancer de la prostate (PCSP*) et éventuellement de la ou des premières et/ou secondes images segmentées (SI₁*, SI₂*), et/ou le stockage du stade local prédit du cancer de la prostate (PCSP*) et éventuellement de la ou des premières et/ou secondes images segmentées (SI₁*, SI₂*) sur une mémoire de données, et/ou la transmission du stade local prédit du cancer de la prostate (PCSP*) et éventuellement de la ou des premières et/ou secondes images segmentées (SI₁*, SI₂*) à un système informatique à distance.
